# EUROPEAN PATENT APPLICATION

(11) **EP 4 818 197 A1**
(43) Date of publication of application: **07.10.2026**
(21) Application number: 25168048.4
(22) Date of filing: 02.04.2025
(51) Int. Cl.: C07K 14/605, B01D 15/38, G01N 33/74

(54) **NOVEL AFFINITY LIGANDS FOR THE PURIFICATION OF DUAL GIP/GLP-1 RECEPTOR AGONISTS**

(71) Applicant: NAVIGO PROTEINS GMBH, 06120 Halle/Saale (DE)
(72) Inventor: BOECKER, Heike, 06120 Halle/Saale (DE); BOBOLOWSKI, Hanna, 06120 Halle/Saale (DE); FIEDLER, Erik, 06120 Halle/Saale (DE); NIEMEYER, Michael, 06120 Halle/Saale (DE)
(74) Representative: Haucke, Kerstin

(57) **Abstract**

The present invention relates to novel affinity ligands that specifically bind to an agonist for glucose-dependent insulinotropic polypeptide (GIP) and glucagon-like peptide 1 (GLP-1), in particular dual GIP/GLP-1 receptor agonists corresponding to Tirzepatide. The present invention further relates to the use of the novel affinity ligands in chromatographic purification processes of such GIP/GLP-1 receptor agonists. The novel affinity ligands of the present invention are powerful tools because they can be used in one-step purification methods for GIP/GLP-1 receptor agonists via affinity chromatography.

## Description

### TECHNICAL FIELD

The present invention relates to novel affinity ligands that specifically bind to an agonist for glucose-dependent insulinotropic polypeptide (GIP) and glucagon-like peptide 1 (GLP-1), in particular dual GIP/GLP-1 receptor agonists corresponding to Tirzepatide. The present invention further relates to the use of the novel affinity ligands in chromatographic purification processes of such GIP/GLP-1 receptor agonists. The novel affinity ligands of the present invention are powerful tools because they can be used in one-step purification methods for GIP/GLP-1 receptor agonists via affinity chromatography.

### BACKGROUND OF THE INVENTION

Agonists for glucose-dependent insulinotropic polypeptide (GIP) and glucagon-like peptide 1 (GLP-1), in particular dual GIP/GLP-1 receptor agonists corresponding to Tirzepatide, represent therapeutic agents used for the treatment of type 2 diabetes mellitus and obesity. These compounds are typically produced via biotechnological methods, such as recombinant expression or chemical peptide synthesis. The subsequent purification of such peptide-based active pharmaceutical ingredients is a critical step in the manufacturing process, as specific requirements regarding purity, yield, and selectivity must be met.

Current procedures for the purification of GIP/GLP-1 receptor agonists such as Tirzepatide involve several steps and require further strategies to remove impurities, byproducts, and other contaminants to obtain the product.

Chromatographic techniques are commonly used for the purification of peptide therapeutics, especially to separate the target molecule from impurities or side products. Among these, reversed-phase high-performance liquid chromatography (RP-HPLC) and ion-exchange chromatography (IEC) are standard methods for the purification of GIP/GLP-1 receptor agonists such as Tirzepatide.

However, these current purification processes often involve multiple steps, specialized reagents, and harsh conditions. They can be time-consuming, costly, and difficult to scale. Furthermore, they may result in product loss or degradation during processing. There is thus a continuing need for improved, more efficient, and scalable purification methods for GIP/GLP-1 receptor agonists, in order to meet required specifications.

Currently no commercially available specific affinity ligand for the purification of GIP/GLP-1 receptor agonists such as Tirzepatide is known. There is therefore a need in the art for affinity ligands that enable selective, efficient, and robust purification of GIP/GLP-1 receptor agonists.

The present invention meets needs in the art by providing novel affinity ligands for GIP/GLP-1 receptor agonists, in particular dual GIP/GLP-1 receptor agonists corresponding to Tirzepatide.

The above overview does not necessarily describe all technical problems solved by the present invention.

### SUMMARY OF THE INVENTION

A technical challenge in the manufacturing of glucose-dependent insulinotropic polypeptide (GIP) and glucagon-like peptide 1 (GLP-1) receptor agonists, in particular dual GIP/GLP-1 receptor agonists corresponding to Tirzepatide, lies in the efficient and selective separation of the target molecule from structurally related by-products, impurities, or folding isomers, particularly in large-scale production processes. Current reversed-phase high-performance liquid chromatography (RP-HPLC) and ion-exchange chromatography (IEC) have several disadvantages like multiple steps, specialized reagents, and harsh conditions.

Accordingly, it is an object of the present invention to provide affinity ligands for the specific binding of GIP/GLP-1 receptor agonists, in particular dual GIP/GLP-1 receptor agonists corresponding to Tirzepatide, enabling a robust, efficient, and industrially scalable purification of these active compounds. The ligands should exhibit high binding affinity and selectivity towards such GIP/GLP-1 receptor agonists and be suitable for immobilization on solid-phase materials. In addition, they should allow for simplified purification workflows, ideally reducing the number of required purification steps.

This objective is achieved by providing the novel affinity ligands of the present invention that enable a purification of (dual) GIP/GLP-1 receptor agonists via affinity chromatography, in particular a one-step purification of (dual) GIP/GLP-1 receptor agonists via affinity chromatography. The invention further relates to corresponding solid-phase materials comprising the novel ligands, purification methods using the ligands, and uses thereof in the downstream processing of GIP/GLP-1 receptor agonists.

The ligands disclosed herein allow for highly selective capture and elution of (dual) GIP/GLP-1 receptor agonists under scalable conditions. The ligands disclosed herein exhibit advantageous binding performance, operational stability, and reusability, and they significantly reduce the complexity and cost of downstream purification processes.

The present invention provides in particular the following items [1] to [12], without being specifically limited thereto.
[1] An affinity ligand for a dual glucose-dependent insulinotropic polypeptide (GIP) and glucagon-like peptide 1 (GLP-1) receptor agonist, comprising the amino acid sequence of SEQ ID NO: 1, or a sequence with at least 90 % sequence identity thereto, wherein the ligand exhibits a specific binding affinity for a dual GIP/GLP-1 receptor agonist.
[2] The affinity ligand of [1], wherein the dual GIP/GLP-1 receptor agonist comprises the amino acid sequence of SEQ ID NO: 6, or a sequence with at least 90 % amino sequence identity thereto.
[3] A fusion protein comprising: (a) an affinity ligand for a dual GIP/GLP-1 receptor agonist of [1] or [2]; and (b) a protein having at least 90 % sequence identity to SEQ ID NO: 4 and having no detectable binding affinity for a dual GIP/GLP-1 receptor agonist. The binding affinity can be, or preferably is, determined by SPR.
[4] The fusion protein of [3], which has at least 90 % sequence identity to SEQ ID NO: 2.
[5] The fusion protein of [3], which has at least 90 % sequence identity to SEQ ID NO: 3.
[6] The affinity ligand of [1] or [2], or the fusion protein of any one of [3] to [5], which has a binding affinity for a dual GIP/GLP-1 receptor agonist of less than 500 nM, preferably a binding affinity for a dual GIP/GLP-1 receptor agonist comprising the amino acid sequence of SEQ ID NO: 6 of less than 500 nM. The binding affinity can be, or preferably is, determined by SPR.
[7] A solid phase support, or an affinity separation matrix, comprising or having immobilized thereon, the affinity ligand for a dual GIP/GLP-1 receptor agonist of any one of [1], [2], and [6], or the fusion protein of any one of [3] to [6].
[8] Use of the affinity ligand for a GIP/GLP-1 receptor agonist of any one of [1], [2], and [6], or the fusion protein according to any one of [3] to [6], or the solid phase support or affinity separation matrix of [7], in (a) affinity purification of a dual GIP/GLP-1 receptor agonist; (b) sample analysis for the detection of a dual GIP/GLP-1 receptor agonist; or (c) detection methods for the detection of a dual GIP/GLP-1 receptor agonist.
[9] A polynucleotide encoding the affinity ligand for a dual GIP/GLP-1 receptor agonist of any one of [1], [2], and [6], or the fusion protein of any one of [3] to [6].
[10] A method of isolating or purifying a dual glucose-dependent insulinotropic polypeptide (GIP) and glucagon-like peptide 1 (GLP-1) receptor agonist from a liquid, the method comprising: (a) contacting the affinity ligand for a dual GIP/GLP-1 receptor agonist of any one of [1], [2], and [6], or the fusion protein of any one of [3] to [6], with a liquid containing a dual GIP/GLP-1 receptor agonist; (b) separating the dual GIP/GLP-1 receptor agonist bound in step (a) to the affinity ligand for a dual GIP/GLP-1 receptor agonist, or to the fusion protein, respectively, from the liquid; (c) detaching or eluting said dual GIP/GLP-1 receptor agonist from the affinity ligand for a dual GIP/GLP-1 receptor agonist, or the fusion protein, respectively.
[11] A method of isolating or purifying a dual glucose-dependent insulinotropic polypeptide (GIP) and glucagon-like peptide 1 (GLP-1) receptor agonist from a liquid, the method comprising: (a) contacting the solid phase support or affinity separation matrix of [7] with a liquid containing a dual GIP/GLP-1 receptor agonist, thereby binding of a dual GIP/GLP-1 receptor agonist in the liquid to the solid phase support or affinity separation matrix; (b) detaching or eluting said dual GIP/GLP-1 receptor agonist from the solid phase support or affinity separation matrix, respectively.
[12] A method of determining the presence of a dual glucose-dependent insulinotropic polypeptide (GIP) and glucagon-like peptide 1 (GLP-1) receptor agonist in a liquid, the method comprising: (a) contacting the affinity ligand for a dual GIP/GLP-1 receptor agonist of any one of [1], [2], and [6], or the fusion protein of any one of [3] to [6], or the solid phase support or affinity separation matrix of [7], with a liquid containing a dual GIP/GLP-1 receptor agonist; (b) determining the dual GIP/GLP-1 receptor agonist bound in step (a) to the affinity ligand for a dual GIP/GLP-1 receptor agonist, or the fusion protein, or the solid phase support or affinity separation matrix, respectively; (c) optionally detaching or eluting said dual GIP/GLP-1 receptor agonist from the affinity ligand for a dual GIP/GLP-1 receptor agonist, or the fusion protein, or the solid phase support or affinity separation matrix, respectively, prior to determining the dual GIP/GLP-1 receptor agonist.

This summary of the invention is not limiting, and other aspects and embodiments of the invention will become evident from the following description, examples and drawings.

### BRIEF DESCRIPTION OF THE FIGURES.

**FIGURE 1****:** shows binding of 232902 (SEQ ID NO: 3) to Tirzepatide. Analysis via label-free interaction assays using the Sierra SPR-32 system (Bruker). Change in refractive index measured in real time and plotted as response or resonance unit [RU] versus time [sec]. Shown are curves of immobilized 232902 vs Tirzepatide in concentrations of 2,000 nM to 3 nM.
**FIGURE 2****:** shows the elution of Tirzepatide from the affinity matrix with immobilized 232902 (SEQ ID NO: 3) using different pH values.

### DETAILED DESCRIPTION OF THE INVENTION

The present invention provides novel affinity ligands for GIP/GLP-1 receptor agonists such as the peptide Tirzepatide and related applications, in particular in the purification of GIP/GLP-1 receptor agonists. The novel affinity ligands exhibit a surprisingly high affinity for GIP/GLP-1 receptor agonists, and are particularly suitable for an affinity purification of GIP/GLP-1 receptor agonists such as Tirzepatide.

The present invention allows the (production and) purification of biologically active (dual) GIP/GLP-1 receptor agonists, in particular biologically active dual GIP/GLP-1 receptor agonists corresponding to Tirzepatide. The novel affinity ligands for dual GIP/GLP-1 receptor agonists allow an industrially applicable, simple, cost effective, robust and highly efficient process of purification of dual GIP/GLP-1 receptor agonists. Without wishing to be bound by theory, the present invention is considered to facilitate the (production and) purification of dual GIP/GLP-1 receptor agonists (such as Tirzepatide) on an industrial scale with a level of purity sufficient for technical applications and/or therapeutic use.

The present invention provides a solid phase support, or an affinity separation matrix, comprising or having immobilized thereon, a novel affinity ligand for a dual GIP/GLP-1 receptor agonist disclosed herein. With the novel affinity ligands, the present invention enables affinity chromatography supports (resins) specifically optimized for the purification of dual GIP/GLP-1 receptor agonists (such as Tirzepatide). These peptides are used as incretin mimetics in the treatment of metabolic disorders such as type 2 diabetes and obesity. Tirzepatide is such an active substance of a medicine, in particular of a diabetes medicine. Tirzepatide is an International non-proprietary name (INN; INN-tirzepatide).

In contrast to conventional reversed-phase purification methods, which typically require multiple steps and harsh organic solvents, the novel affinity ligands described herein enable a single-step, aqueous-based purification of dual GIP/GLP-1 receptor agonists like Tirzepatide. In particular, the solid phase supports or affinity separation matrices described herein, more specifically chromatography resins described herein, enable a one-step purification process that is considered to offer advantageous performance in terms of impurity removal, product recovery, and overall yield.

The novel affinity ligands of the present invention demonstrate high binding specificity towards dual GIP/GLP-1 receptor agonists, facilitating efficient separation from structurally related impurities, degradation products, or synthesis by-products.

The solid phase supports or affinity separation matrices of the invention as described herein, in particular (affinity) chromatography resins of the invention described herein, are considered to exhibit advantageous scalability and robustness, allowing for seamless integration from pilot-scale to commercial manufacturing processes. It is considered to withstand repeated use under caustic cleaning conditions, and is thus considered to ensure long-term operational stability and cost-effectiveness.

In comparison to conventional multi-step purification protocols, the use of the affinity matrices of the present invention are considered to significantly reduce the number of required process steps, solvent consumption, and buffer volumes. As a result, cost of goods can be minimized while maintaining high product purity and compliance with regulatory standards.

The novel affinity ligands of the present invention are considered high-performance affinity ligands for dual GIP/GLP-1 receptor agonists. The affinity matrices of the present invention are considered to be compatible with current good manufacturing practice (cGMP) environments and are considered to support regulatory-compliant biomanufacturing, making it well-suited for accelerated development and commercialization pathways.

The present invention is considered to be particularly useful in the downstream processing of incretin mimetic peptides by offering a scalable, robust, and economically viable one-step purification solution.

The present invention provides affinity ligands and fusion proteins for the specific binding and separation or purification of dual GIP/GLP-1 receptor agonists. The ligands comprise SEQ ID NO: 1, or a sequence having at least 90 % sequence identity thereto, and exhibit specific binding affinity for dual GIP/GLP-1 receptor agonists such as Tirzepatide (SEQ ID NO: 6 is the amino acid sequence underlying Tirzepatide).

In certain embodiments, the ligand is part of a fusion protein. The fusion proteins similarly enable selective binding and separation or purification of GIP/GLP-1 receptor agonists and can be immobilized on solid-phase supports as described elsewhere herein, such as agarose, sepharose, or synthetic matrices.

The present invention provides fusion proteins comprising a novel affinity ligand for a dual GIP/GLP-1 receptor agonist as disclosed herein and a protein (peptide), which has at least 90 % sequence identity to SEQ ID NO: 4 and which has no detectable binding affinity for a dual GIP/GLP-1 receptor agonist, in particular a dual GIP/GLP-1 receptor agonist as described herein. The present invention also provides fusion proteins comprising a novel affinity ligand for a dual GIP/GLP-1 receptor agonist as disclosed herein and a protein (peptide), which has at least 90 % sequence identity to SEQ ID NO: 5 and which has no detectable binding affinity for a dual GIP/GLP-1 receptor agonist, in particular a dual GIP/GLP-1 receptor agonist as described herein. The non-dual GIP/GLP-1 receptor agonist binding proteins (peptides) are considered to enhance spatial orientation, reduce steric hindrance, and potentially improve the accessibility of the affinity ligand domain when immobilized. The peptide having at least 90 % sequence identity to SEQ ID NO: 4 or SEQ ID NO: 5 and having no detectable binding affinity for a dual GIP/GLP-1 receptor agonist is also a peptide that does not interact with the Fc region (or Fc domain) of immunoglobulins (i.e., is a non-Fc-binding peptide).

Provided herein are fusion proteins, in which the affinity ligand sequence is located at the N-terminus, and a non-Fc-binding protein sequence is fused to the C-terminus. This C-terminal component has no detectable binding affinity for dual GIP/GLP-1 receptor agonists, in particular Tirzepatide, and does not interact with the Fc region of immunoglobulins. Two variants of this component are described herein in SEQ ID NO: 4 and SEQ ID NO: 5, which differ only in the presence or absence of two amino acids at the N-terminus. Accordingly, fusion proteins specifically provided herein have at least 90 % sequence identity to SEQ ID NO: 2. SEQ ID NO: 2 comprises SEQ ID NO: 1 fused to SEQ ID NO: 5.

Fusion proteins according to SEQ ID NO: 2 are preferred embodiments of the present invention. Fusion proteins having at least 90 % sequence identity to SEQ ID NO: 2 are characterized in that the affinity ligand for a dual GIP/GLP-1 receptor agonist comprised by the fusion protein exhibits a specific binding affinity for a dual GIP/GLP-1 receptor agonist (as described elsewhere herein), and the non-Fc-binding peptide fused to the C-terminus has no detectable binding affinity for a dual GIP/GLP-1 receptor agonist and does not interact with the Fc region of immunoglobulins.

Fusion proteins according to SEQ ID NO: 3 are particularly preferred embodiments of the present invention. SEQ ID NO: 3 contains tandem duplications of the non-Fc-binding domain (i.e., two copies of SEQ ID NO: 5) at the C-terminus. Fusion proteins as reflected by SEQ ID NO: 3 represent extended constructs, each comprising SEQ ID NO: 1 fused to two consecutive copies of SEQ ID NO: 5. These tandem-repeat constructs are considered to enhance spatial orientation, reduce steric hindrance, and potentially improve the accessibility of the ligand domain when immobilized.

Fusion proteins having at least 90 % sequence identity to SEQ ID NO: 3 are characterized in that the affinity ligand for a dual GIP/GLP-1 receptor agonist comprised by the fusion protein exhibits a specific binding affinity for a dual GIP/GLP-1 receptor agonist (as described elsewhere herein), and the non-Fc-binding peptide fused to the C-terminus has no detectable binding affinity for a dual GIP/GLP-1 receptor agonist and does not interact with the Fc region of immunoglobulins.

The novel affinity ligands of the present invention, and the fusion proteins of the present invention, have a specific binding affinity for dual GIP/GLP-1 receptor agonists of less than 500 nM. More specifically, the novel affinity ligands of the present invention, or the fusion proteins of the present invention, have a specific binding affinity for dual GIP/GLP-1 receptor agonists described herein in relation to SEQ ID NO: 6 and variant dual GIP/GLP-1 receptor agonists described herein in relation to SEQ ID NO: 6, of less than 500 nM. Even more specifically, the novel affinity ligands of the present invention, or the fusion proteins of the present invention, have a specific binding affinity for Tirzepatide of less than 500 nM.

Preferably, the novel affinity ligands of the present invention, or the fusion proteins of the present invention, have a specific binding affinity for dual GIP/GLP-1 receptor agonists of less than 400 nM, or even less than 350 nM. More specifically, the novel affinity ligands of the present invention, or the fusion proteins of the present invention, have a specific binding affinity for dual GIP/GLP-1 receptor agonists described herein in relation to SEQ ID NO: 6 and variant dual GIP/GLP-1 receptor agonists described herein in relation to SEQ ID NO: 6, of less than 400 nM, or even less than 350 nM. Even more specifically, the novel affinity ligands of the present invention, or the fusion proteins of the present invention, have a specific binding affinity for Tirzepatide of less than 400 nM, or even less than 350 nM.

The novel affinity ligand of the present invention according to SEQ ID NO: 1, and the corresponding fusion proteins of the present invention (SEQ ID NO: 2 and SEQ ID NO: 3), do not exhibit detectable binding affinity for the GLP-1 receptor agonist Semaglutide (INN-semaglutide). In certain or preferred embodiments, variants of SEQ ID NO: 1 encompassed by the present invention, and corresponding fusion proteins of the present invention, do likewise not exhibit a detectable binding affinity for the GLP-1 receptor agonist Semaglutide.

Before the present invention is described in more detail below, it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is reflected by the appended items. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of binding molecules for dual GIP/GLP-1 receptor agonists and their use as affinity ligands in the purification of Tirzepatide in affinity chromatography.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the items, which follow, unless the context requires otherwise, the word "comprise", and variants such as "comprises" and "comprising", will be understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

Several documents (for example: patents, patent applications, scientific publications, manufacturer's specifications, instructions, UniProt Accession Number, etc.) may be cited throughout the present specification. Nothing herein is to be construed as an admission that the invention is not entitled to antedate such disclosure by virtue of prior invention. Some of the documents cited herein may be characterized as being *"incorporated by reference".* In the event of a conflict between the definitions or teachings of such incorporated references and definitions or teachings recited in the present specification, the text of the present specification takes precedence.

All sequences referred to herein are disclosed in the attached sequence listing that, with its whole content and disclosure, forms part of the disclosure content of the present specification.

### General Definitions of Important terms used in the Application

The incretins glucose-dependent insulinotropic polypeptide (GIP) and glucagon-like peptide 1 (GLP-1) are known to mediate insulin responses that are proportionate to nutrient intake to facilitate glucose tolerance. The glucose-dependent insulinotropic polypeptide (GIP) is also known as gastric inhibitory polypeptide (GIP). Both terms may be used interchangeably herein.

The terms "(dual) GIP/GLP-1 receptor agonist" and "(dual) GIP and GLP-1 receptor agonist" may be used interchangeably herein. It will be appreciated that the term "(dual) GIP and GLP-1 receptor agonist" is intended to refer to "(dual) GIP receptor and GLP-1 receptor agonist".

The term "(dual) GIP/GLP-1 receptor agonist" as used herein includes Tirzepatide (INN-tirzepatide). Tirzepatide is an analog of the human GIP hormone. Tirzepatide is an agonist of both the GIP receptor and the GLP-1 receptor. Accordingly, dual GIP/GLP-1 receptor agonists encompassed by the present invention described elsewhere herein are agonists of both the GIP receptor and the GLP-1 receptor. Tirzepatide may be considered a twincretin, acting on both GLP-1 and GIP receptors. Accordingly, dual GIP/GLP-1 receptor agonists encompassed by the present invention described elsewhere herein may be considered twincretins, acting on both GLP-1 and GIP receptors. Dual GIP/GLP-1 receptor agonists encompassed by the present invention described herein may also be considered single-molecule co-agonists for the glucagon-like peptide-1 (GLP-1) receptor and the glucose-dependent insulinotropic polypeptide (GIP) receptor.

As described herein, the terms "(dual) GIP/GLP-1 receptor agonist", "(dual) GIP (receptor) and GLP-1 receptor agonist", and "single-molecule co-agonists for the GIP (receptor) and GLP-1 receptor" may be used interchangeably herein.

The dual GIP/GLP-1 receptor agonists described herein can be used in GIP receptor-GLP-1 receptor co-agonism to decrease body weight with an efficacy, which may be superior relative to GLP-1 receptor agonism alone.

The term "(dual) GIP/GLP-1 receptor agonist" comprises all polypeptides, which show an amino acid sequence identity of at least 80 %, 90 %, 95 %, 96 % or 97 % or more, or 100 % to SEQ ID NO: 6. In some embodiments, the term "dual GIP/GLP-1 receptor agonist" refers to a peptide comprising an amino acid sequence with at least 90 % identity to the amino acid sequence of SEQ ID NO: 6. In some embodiments, the term "dual GIP/GLP-1 receptor agonist" refers to a peptide comprising an amino acid sequence with at least 95 % identity to the amino acid sequence of SEQ ID NO: 6. In preferred embodiments, the term "dual GIP/GLP-1 receptor agonist" refers to a peptide comprising an amino acid sequence with at least 98 % identity to the amino acid sequence of SEQ ID NO: 6.

The amino acid sequence of Tirzepatide (SEQ ID NO: 6) has an alpha-aminoisobutyric acid (Aib) at the positions corresponding to positions 2 and 13 of SEQ ID NO: 6; and has a fatty (di)acid moiety, in particular a C20 fatty (di)acid chain (eicosanedioic acid), more specifically a C20 fatty (di)acid (L-)γ-glutamic acid moiety, attached (linked) to the amino acid at the position corresponding to position 20 of SEQ ID NO: 6. Preferably, the amino acid at position 20 of SEQ ID NO: 6 is a lysine residue (Lys).

The structural modification at the amino acid at position 20 of SEQ ID NO: 6 includes a connection to the (C20) fatty (di)acid moiety at the amino acid residue at position 20 of SEQ ID NO: 6 through a linker. Specifically, the linker is or contains a hydrophilic spacer. The linker is composed of an L-γ-glutamic acid and two 8-amino-3,6-dioxaoctanoic acids (γ-Glu(AEEA)₂).

In preferred embodiments of the present invention, the term "dual GIP/GLP-1 receptor agonist" comprises peptides, which have an amino acid sequence identity of at least 80 %, 90 %, 90 %, 95 %, 96 % or 97 % or more, or 100 % to SEQ ID NO: 6, and which comprise (i) an alpha-aminoisobutyric acid (Aib) at the positions corresponding to positions 2 and 13 of SEQ ID NO: 6; (ii) a lysine residue (Lys) at the position corresponding to position 20 of SEQ ID NO: 6; and/or (iii) a fatty acid moiety, in particular a fatty (di)acid chain, attached (linked) to the amino acid at the position corresponding to position 20 of SEQ ID NO: 6. Preferably, the fatty (di)acid chain is a C-20 fatty (di)acid chain and/or the amino acid at the position corresponding to position 20 of SEQ ID NO: 6 is lysine (Lys). The fatty (di)acid chain is in particular a C20 fatty (di)acid (L-)γ-glutamic acid moiety. Still more preferably, the fatty (di)acid chain is attached (linked) to the amino acid at the position corresponding to position 20 of SEQ ID NO: 6 via a hydrophilic spacer (linker), preferably via a (L-)γ-glutamic acid moiety and a hydrophilic spacer. The hydrophilic spacer can be made of two 8-amino-3,6-dioxaoctanoic acid ((2-(2-aminoethoxy)ethoxy)acetic acid; AEEA) units.

Accordingly, variants of the "dual GIP/GLP-1 receptor agonist" of SEQ ID NO: 6 described herein preferably have a fatty (di)acid moiety at the amino acid position corresponding to position 20 of SEQ ID NO: 6, wherein the fatty (di)acid moiety is preferably attached via a hydrophilic spacer (or linker), more preferably via a hydrophilic spacer (or linker) and a (L-)γ-glutamic acid moiety. Preferably, the hydrophilic spacer/linker is made of two amino-ethoxy ethoxy acetic acid ((2-(2-aminoethoxy)ethoxy)acetic acid; AEEA) units. In preferred embodiments, the fatty (di)acid moiety is a C20 fatty (di)acid chain. In particularly preferred embodiments, the derivatization at the amino acid corresponding to position 20 of SEQ ID NO: 6 consists of two 8-amino-3,6-dioxaoctanoic acid (ADO) spacers, one γ-glutamic acid (Glu), and a fatty diacid, which is C20 eicosanedioic acid. It is preferred that variants of the "dual GIP/GLP-1 receptor agonist" of SEQ ID NO: 6 described herein have a lysine residue (Lys) at the position corresponding to position 20 of SEQ ID NO: 6.

In preferred embodiments, the term "dual GIP/GLP-1 receptor agonist" refers to an amino acid sequence with at least 98 % identity to the amino acid sequence of SEQ ID NO: 6 and comprising the aforementioned modifications at the positions corresponding to positions 2, 13, and/or 20 of SEQ ID NO: 6.

The C-terminus of Tirzepatide is amidated . Accordingly, in certain or even preferred embodiments of the present invention, variant dual GIP/GLP-1 receptor agonists described herein in relation to SEQ ID NO: 6 contain an amidated C-terminus, in particular at the C-terminal amino acid residue located at the position corresponding to position 39 of SEQ ID NO: 6.

As described herein, dual GIP/GLP-1 receptor agonists encompassed by the present invention may exhibit the same biological function or activity as a dual GIP/GLP-1 receptor agonist comprising the amino acid sequence of SEQ ID NO: 6. In preferred embodiments, dual GIP/GLP-1 receptor agonists encompassed by the present invention may exhibit the same biological function or activity as Tirzepatide. More specifically, variants of the dual GIP/GLP-1 receptor agonist of SEQ ID NO: 6 described herein may exhibit the same biological function or activity as a dual GIP/GLP-1 receptor agonist comprising the amino acid sequence of SEQ ID NO: 6. In preferred embodiments, variants of the dual GIP/GLP-1 receptor agonist of SEQ ID NO: 6 described herein may exhibit the same biological function or activity as Tirzepatide.

As described herein, the (same) biological function or activity of a dual GLP-1 receptor agonist comprising the amino acid sequence of SEQ ID NO: 6, or the (same) biological function or activity of Tirzepatide, is (binding to and) activating the GIP and GLP-1 receptors. Accordingly, the biological function or activity of a dual GLP-1 receptor agonist comprising the amino acid sequence of SEQ ID NO: 6, or the biological function or activity of Tirzepatide, is a dual agonist activity for the GIP and GLP-1 receptors.

Tirzepatide is described as the only dual GIP/GLP-1 receptor agonist that can reduce glycemic levels and improve insulin sensitivity.

Tirzepatide is described to stimulate insulin secretion in the human pancreas via the GIP receptor and to reduce glucagon levels. Tirzepatide is also described as a peptide with glucose-lowering effects.

As described herein, the activation of the GIP and GLP-1 receptors is considered to stimulate insulin secretion and reduce blood glucose. Accordingly, the (same) biological function or activity of a dual GLP-1 receptor agonist comprising the amino acid sequence of SEQ ID NO: 6, or the (same) biological function or activity of Tirzepatide, as described herein may be determined by measuring (a modulation of) insulin secretion, or measuring (a modulation of) insulin level secretion, in particular an increase in insulin secretion or insulin level; and/or measuring (a modulation of) blood glucose (level), in particular a reduction of blood glucose (level). The said modulation of insulin secretion / insulin level or blood glucose (level) can be determined against the modulation of insulin secretion / insulin level or blood glucose (level), respectively, in the absence of the GIP/GLP-1 receptor agonist.

In certain or even preferred embodiments, the (same) biological function or activity of a dual GLP-1 receptor agonist comprising the amino acid sequence of SEQ ID NO: 6, or the (same) biological function or activity of Tirzepatide, may be superior relative to GLP-1 receptor agonism alone. Accordingly, in certain or even preferred embodiments, the (same) biological function or activity of a dual GLP-1 receptor agonist comprising the amino acid sequence of SEQ ID NO: 6, or the (same) biological function or activity of Tirzepatide, may be superior relative to the GLP-1 receptor agonist Semaglutide (INN-semaglutide).

As described herein, the (same) biological function or activity means the (same) biological function or activity under the same conditions. More specifically, the (same) biological function or activity means the (same) biological function or activity as determined under the same conditions.

Accordingly, the present invention allows the (production and) purification of biologically active dual GIP/GLP-1 receptor agonists, in particular dual GIP/GLP-1 receptor agonists corresponding to Tirzepatide.

Without wishing to be bound by theory, the present invention allows the (production and) purification of dual GIP/GLP-1 receptor agonists, in particular dual GIP/GLP-1 receptor agonists corresponding to Tirzepatide, while (at least) maintaining the biological function or activity of Tirzepatide and/or the native GIP/GLP-1 receptor agonists.

As described herein, a dual GIP/GLP-1 receptor agonist corresponding to Tirzepatide as described herein is considered to encompass variant dual GIP/GLP-1 receptor agonists described elsewhere herein in relation to SEQ ID NO: 6, in particular variant dual GIP/GLP-1 receptor agonists described elsewhere herein in relation to SEQ ID NO: 6 that comprise the aforementioned modifications at the positions corresponding to positions 2, 13, and/or 20 of SEQ ID NO: 6.

The affinity ligands and fusion proteins described herein provide multiple technical advantages over conventional purification strategies for dual GIP/GLP-1 receptor agonists, such as Tirzepatide. In contrast to established methods based on reversed-phase or ion-exchange chromatography, which often require multiple sequential steps, organic solvents, or complex buffer systems, the present invention enables one-step purification under aqueous conditions.

The methods of the present invention may be considered as one-step methods as compared to dual GIP/GLP-1 receptor agonist (or GLP-1 receptor agonist) separation/purification methods established in the art, which typically require multiple steps, as described herein above in relation to conventional reversed-phase purification methods. Without wishing to be bound by theory, the present invention is the first to provide an affinity chromatography-based method for isolation, purification and/or separation of dual GIP/GLP-1 receptor agonists. Accordingly, without wishing to be bound by theory, the present invention is the first to provide a one-step method for isolation, purification and/or separation of dual GIP/GLP-1 receptor agonists, in particular a one-step affinity chromatography method for isolation, purification or separation of dual GIP/GLP-1 receptor agonists. Accordingly, in the methods of the present invention, the solid phase support or affinity separation matrix is preferably an affinity chromatography support, or an affinity chromatography matrix. The terms affinity chromatography support, affinity chromatography matrix, and affinity chromatography column may be used interchangeably herein.

The novel affinity ligands of the present invention exhibit binding affinities in the sub-micromolar range, i.e., in the nanomolar range, which is considered to be stronger than nonspecific interactions used in traditional chromatographic techniques. This high specificity allows for greater impurity clearance, improved recovery, and significantly reduced processing time and buffer consumption. Furthermore, the fusion constructs of the present invention are considered to demonstrate advantageous chemical and mechanical stability.

Taken together, the novel affinity ligands of the present invention, and the novel affinity matrices accordingly, provide for advantageous properties in the downstream processing of incretin mimetic peptides, in particular in terms of process simplicity, product quality, and industrial applicability.

The term "fusion protein" relates to a protein comprising at least a first protein joined genetically to at least a second protein. A fusion protein is created through joining of two or more genes that originally coded for separate proteins. Thus, a fusion protein may comprise a multimer of identical or different proteins which are expressed as a single, linear polypeptide.

The present invention encompasses a fusion protein comprising an affinity ligand for GIP/GLP-1 receptor agonist as described above fused to at least one further protein (peptide) as described elsewhere herein.

The terms "affinity ligand" or "binding protein" may be used interchangeably herein and describe a protein that is capable to bind to (and activate) a dual GIP/GLP-1 receptor agonist. As described herein, a binding protein refers to a protein with detectable interaction with a dual GIP/GLP-1 receptor agonist, as determined by suitable methods such as for example SPR (surface plasmon resonance) analysis or BLI (bio-layer interferometry) or any other appropriate technology known to a person skilled in the art.

The term "binding affinity" refers to the ability of the proteins of the invention to bind to (and activate) a dual GIP/GLP-1 receptor agonist.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the amino acid sequence of the polypeptide of SEQ ID NO: 1. Methods for sequence alignment are well known in the art. For example, the NCBI BLAST similarity program is preferably employed (Camacho et al., 2009, BMC Bioinformatics 10: 421). For multiple alignment analysis, ClustalL is preferably used (Sievers & Higgins, 2021, Methods Mol Biol. 2231: 3-16). Thus, the percentage of an amino acid sequence identity may be calculated using a published or commercially available software with an algorithm which conducts comparison using a base sequence (e.g., SEQ ID NO: 1 in the present invention) as a reference sequence. For example, BLAST, FASTA, or GENETYX (manufactured by Software Development Co., Ltd.) may be used, and these may be run with default parameters. Each amino acid of the query sequence that differs from the aligned reference amino acid sequence at a given position is counted as one difference. An insertion or deletion in the query sequence is also counted as one difference. The sum of differences is then related to the length of the aligned reference sequence to yield a percentage of non-identity or identity, respectively.

The terms "protein" and "polypeptide" refer to any chain of two or more amino acids linked by peptide bonds and does not refer to a specific length of the product. Thus, "peptides", "protein", "amino acid chain", or any other term used to refer to a chain of two or more amino acids, are included within the definition of "polypeptide", and the term "polypeptide" may be used instead of, or interchangeably with, any of these terms. The term "polypeptide" is also intended to refer to the products of post-translational modifications of the polypeptide like, e.g., glycosylation, which are well known in the art.

A "linker" as used herein is preferably an amino acid sequence that joins at least two moieties, e.g., two affinity ligands of the present invention, or two monomers of a multimeric affinity ligand of the present invention. The linker as used herein is a peptide linker of up to 30 amino acids. Two or more moieties may be linked via a peptide linker of 5, 6, 7, 8, 9,10, 11, 12, 13, 14, 15, 16, 17, 18, 19, 20, 21, 22, 23, 24, 25, 26, 27, 28, 29, or 30 amino acids. In preferred embodiments, a linker comprises two amino acids, in particular isoleucine (I) and alanine (A). In the fusion proteins of the present invention, a protein having at least 90 % sequence identity to SEQ ID NO: 1 and a protein having at least 90 % sequence identity to SEQ ID NOs: 4 or 5 as described elsewhere herein, may also be linked by a linker, in particular a peptide linker.

The term "chromatography" refers to separation technologies, which employ a mobile phase and a stationary phase to separate one type of molecules (e.g., a dual GIP/GLP-1 receptor agonist described herein) from other molecules (e.g., contaminants) in a liquid or sample. The (liquid) mobile phase contains a mixture of molecules and transports these across or through a stationary phase (such as a solid matrix). Due to the differential interaction of the different molecules in the mobile phase with the stationary phase, molecules in the mobile phase can be separated.

The term "affinity chromatography" refers to a specific mode of chromatography in which a ligand (e.g., affinity ligand of the invention) coupled to a stationary phase (e.g. solid support) interacts with a molecule (i.e., a dual GIP/GLP-1 receptor agonist) in the mobile phase (the liquid sample), i.e., the ligand has a specific binding affinity for the molecule to be captured. As understood in the context of the invention, affinity chromatography involves the addition of a liquid or (liquid) sample containing dual GIP/GLP-1 receptor agonists to a stationary phase which comprises a chromatography ligand, such as the affinity ligand of the invention. The terms "solid support" or "solid matrix" are used interchangeably for the stationary phase.

The terms "affinity matrix" or "affinity separation matrix", as used interchangeably herein, refer to a matrix, e.g., a chromatographic matrix, onto which an affinity ligand of the invention is attached. The attached affinity ligand is capable of specific binding to a protein of interest (e.g., a dual GIP/GLP-1 receptor agonist) which is to be purified from a liquid or a (liquid) sample.

The term "affinity purification" as used herein refers to a method of purifying a dual GIP/GLP-1 receptor agonist from a liquid or (liquid) sample by binding dual GIP/GLP-1 receptor agonists to a ligand of the invention that is immobilized to a matrix. Thereby, dual GIP/GLP-1 receptor agonists are removed from the liquid or (liquid) sample and thereby purified.

### Detailed description of the embodiments of the invention

The present invention will be further described in the following. In the following passages different aspects of the invention are defined in more detail. Each aspect defined below may be combined with any other aspect or aspects unless clearly indicated to the contrary. In particular, any feature indicated as being preferred or advantageous may be combined with any other feature or features indicated as being preferred or advantageous.

The novel affinity ligands of the present invention exhibit a specific binding affinity for GIP/GLP-1 receptor agonist. In particular, the invention provides affinity ligands for dual GIP/GLP-1 receptor agonists, comprising the amino acid sequence of SEQ ID NO: 1 or a sequence with at least 90 % sequence identity thereto, preferably with at least 90 % sequence identity thereto.

In a particularly preferred embodiment, the affinity ligand comprises the amino acid sequence of SEQ ID NO: 1. This ligand is incorporated into a fusion protein with SEQ ID NO: 5 as the C-terminal domain, resulting in SEQ ID NO: 2. This fusion protein can be immobilized, e.g., on an agarose matrix and used for the purification of Tirzepatide (SEQ ID NO: 6).

Further preferred embodiments include fusion protein SEQ ID NO: 3, which comprise two tandem repeats of the non-Fc-binding domain (SEQ ID NO: 5). These constructs are considered to exhibit advantageous ligand orientation and binding kinetics, providing for advantageous performance in high-throughput or continuous purification systems.

**Structural characteristics of the affinity ligands.** In some embodiments, the affinity ligand for a dual GIP/GLP-1 receptor agonist (Tirzepatide) comprises the amino acid sequence of SEQ ID NO: 1. In some embodiments, the affinity ligand for a dual GIP/GLP-1 receptor agonist (Tirzepatide) comprises an amino acid with at least 90 % sequence identity to SEQ ID NO: 1. In some embodiments, the affinity ligand for a dual GIP/GLP-1 receptor agonist (Tirzepatide) has at least 90 %, 91 %, 92 %, 93 %, 94 %, 95%, 96 %, 97 %, 98 %, 99 %, or 100 % sequence identity to SEQ ID NO: 1.

**Structural characteristics: Multimers.** Some embodiments refer to an affinity ligand for a dual GIP/GLP-1 receptor agonist (e.g., Tirzepatide), wherein the affinity ligand comprises the amino acid sequence of SEQ ID NO: 1, or an amino acid sequence with at least 90 % sequence identity thereto as described elsewhere herein, wherein the affinity ligand is a multimer. Some embodiments refer to a multimer of the dual GIP/GLP-1 receptor agonist affinity ligand, wherein the affinity ligand comprises the amino acid sequence of SEQ ID NO: 1, or an amino acid sequence with at least 90 % sequence identity thereto as described elsewhere herein, wherein at least two dual GIP/GLP-1 receptor agonist affinity ligands as described above are linked to each other.

Some embodiments refer to a dimer of the dual GIP/GLP-1 receptor agonist affinity ligand, wherein the affinity ligand comprises the amino acid sequence of any one selected from SEQ ID NO: 1, or an amino acid sequence with at least 90 % sequence identity thereto as described elsewhere herein, wherein at least two dual GIP/GLP-1 receptor agonist affinity ligands as described above are linked to each other.

In some embodiments, two dual GIP/GLP-1 receptor agonist affinity ligands comprising the amino acid sequence of SEQ ID NO: 1, or an amino acid sequence with at least 90 % sequence identity thereto as described elsewhere herein, may be directly fused to each other, or may be linked by a peptide linker, as described elsewhere herein.

**Functional characteristics of the affinity ligand.** One of the advantages of the herein disclosed affinity ligands is the specific binding to the dual GIP/GLP-1 receptor agonist Tirzepatide. In specific embodiments, the dual GIP/GLP-1 receptor agonist (Tirzepatide) affinity ligand comprising the amino acid sequence of any one of SEQ ID NO: 1, or a sequence with at least 90 % sequence identity thereto as described elsewhere herein, has a specific binding affinity of less than 500 nM for the dual GIP/GLP-1 receptor agonist Tirzepatide as disclosed herein. In some embodiments, the affinity ligand has a specific binding affinity of less than 400 nM for the dual GIP/GLP-1 receptor agonist Tirzepatide as disclosed herein. In some embodiments, the affinity ligand has a binding affinity of less than 350 nM for the dual GIP/GLP-1 receptor agonist Tirzepatide as disclosed herein.

Binding affinity is typically measured and reported by the equilibrium dissociation constant (K_{D}), which is used to evaluate and rank the strength of bimolecular interactions. The binding affinity and dissociation constants can be measured quantitatively. Methods for determining binding affinities are well known to the skilled person and can be selected, for instance, from the following methods that are well established in the art: surface plasmon resonance (SPR), Bio-layer interferometry (BLI), enzyme-linked immunosorbent assay (ELISA), kinetic exclusion analysis (KinExA assay), flow cytometry, fluorescence spectroscopy techniques, isothermal titration calorimetry (ITC), analytical ultracentrifugation, radioimmunoassay (RIA or IRMA), and enhanced chemiluminescence (ECL). Typically, the dissociation constant K_{D} is determined at temperatures in the range of 20°C and 30°C. If not specifically indicated otherwise, K_{D} values recited herein are determined at 25°C, more specifically at 25°C by SPR. Even more specifically, the binding affinity (K_{D}) is determined by SPR at 25°C at about neutral pH, in particular at pH 7.3. In various embodiments of the present invention, the binding affinity for GIP/GLP-1 receptor agonist (Tirzepatide) may be determined by the Sierra SPR-32 system (Bruker).

**Fusion proteins.** In some embodiments, the invention refers to a fusion protein comprising:
(a) a dual GIP/GLP-1 receptor agonist affinity ligand of the present invention as described herein; and
(b) a protein with at least 90 % amino acid sequence identity to the sequence of SEQ ID NO: 4 (or SEQ ID NO: 5), which has no detectable binding affinity for a dual GIP/GLP-1 receptor agonist, for example, as determined by SPR.

Further, the protein with at least 90 % amino acid sequence identity to the sequence of SEQ ID NO: 4 (or SEQ ID NO: 5) is a protein having no detectable binding affinity for the Fc domain of immunoglobulin, for example, as determined by SPR.

In preferred embodiments, the peptide with at least 90 % amino acid sequence identity to the sequence of SEQ ID NO: 4 is a peptide comprising an acidic amino acid selected from aspartic acid (D) and glutamic acid (E) at the position corresponding to position 11 of SEQ ID NO: 4, and a basic amino acid selected from any one of arginine (R), lysine (K), and histidine (H) at the position corresponding to position 29 of SEQ ID NO: 4, wherein the peptide has no detectable binding affinity for the Fc domain of immunoglobulin, for example, as determined by SPR.

In preferred embodiments, the protein with at least 90 % amino acid sequence identity to the sequence of SEQ ID NO: 4 is a peptide comprising aspartic acid (D) at the position corresponding to position 11 of SEQ ID NO: 4, and arginine (R) at the position corresponding to position 29 of SEQ ID NO: 4, wherein the peptide has no detectable binding affinity for the Fc domain of immunoglobulin, for example, as determined by SPR.

In preferred embodiments, the peptide with at least 90 % amino acid sequence identity to the sequence of SEQ ID NO: 5 is a peptide comprising an acidic amino acid selected from aspartic acid (D) and glutamic acid (E) at the position corresponding to position 13 of SEQ ID NO: 5, and a basic amino acid selected from any one of arginine (R), lysine (K), and histidine (H) at the position corresponding to position 31 of SEQ ID NO: 5, wherein the peptide has no detectable binding affinity for the Fc domain of immunoglobulin, for example, as determined by SPR.

In preferred embodiments, the protein with at least 90 % amino acid sequence identity to the sequence of SEQ ID NO: 5 is a peptide comprising aspartic acid (D) at the position corresponding to position 13 of SEQ ID NO: 5, and arginine (R) at the position corresponding to position 31 of SEQ ID NO: 5, wherein the peptide has no detectable binding affinity for the Fc domain of immunoglobulin, for example, as determined by SPR.

In some embodiments, the invention refers to a fusion protein comprising:
(a) a dual GIP/GLP-1 receptor agonist affinity ligand of the present invention as described herein; and
(b) a protein of SEQ ID NO: 4, which has no detectable binding affinity for a dual GIP/GLP-1 receptor agonist, for example, as determined by SPR.

In some embodiments, the invention refers to a fusion protein comprising:
(a) a dual GIP/GLP-1 receptor agonist affinity ligand of the present invention as described herein (SEQ ID NO: 1); and
(b) a protein of SEQ ID NO: 5, which has no detectable binding affinity for a dual GIP/GLP-1 receptor agonist, for example, as determined by SPR.

In some embodiments, the invention refers to a dual GIP/GLP-1 receptor agonist affinity ligand of the present invention as described herein, or a multimeric dual GIP/GLP-1 receptor agonist affinity ligand of the present invention as described herein, or a fusion protein of the present invention as described herein, which has a specific binding affinity for the dual GIP/GLP-1 receptor agonist Tirzepatide of less than 500 nM, as determined by SPR.

In some embodiments, the fusion protein of the present invention has a specific binding affinity of less than 500 nM for the dual GIP/GLP-1 receptor agonist Tirzepatide as described herein. In preferred embodiments, the fusion protein of the present invention has a specific binding affinity of less than 400 nM for the dual GIP/GLP-1 receptor agonist Tirzepatide as described herein.

In other preferred embodiments, the fusion protein of the present invention has a specific binding affinity of less than 350 nM for the dual GIP/GLP-1 receptor agonist Tirzepatide as described herein.

In some embodiments, the fusion protein of the present invention has no detectable binding affinity for the GLP-1 receptor agonist Semaglutide.

Some embodiments refer to a fusion protein comprising the amino acid sequence of any one of SEQ ID NO: 2 or SEQ ID NO: 3, or a sequence with at least 90 % sequence identity thereto, respectively, as described elsewhere herein.

In one embodiment, additional amino acid sequences include one or more peptide sequences that confer an affinity to certain chromatography column materials. The affinity ligand or the fusion protein may include specific attachment sites for the attachment to solid supports, preferably at the C-terminal end, such as cysteine or lysine.

**Affinity separation matrix.** One embodiment of the invention refers to an affinity separation matrix comprising an affinity ligand for the dual GIP/GLP-1 receptor agonist (Tirzepatide) of the present invention as described herein, or a fusion protein of the present invention as described herein. One particular embodiment refers to such an affinity separation matrix wherein the dual GIP/GLP-1 receptor agonist (Tirzepatide) affinity ligand or the fusion protein comprises the amino acid sequence of SEQ ID NO: 1 or an amino acid sequence with at least 90 % sequence identity thereto, as described elsewhere herein.

In preferred embodiments, the affinity separation matrix is a solid support. The affinity separation matrix comprises at least one affinity ligand for a dual GIP/GLP-1 receptor agonist such as Tirzepatide of the present invention or a fusion protein as described herein.

Solid support matrices for affinity chromatography are known in the art and include, e.g., without being limited thereto, agarose and stabilized derivatives of agarose, cellulose or derivatives of cellulose, controlled pore glass, monolith, silica, zirconium oxide, titanium oxide, or synthetic polymers, and hydrogels of various compositions and combinations of the above. The formats for solid support matrices can be of any suitable well-known kind. Such solid support matrix for coupling a novel affinity ligand of the present invention might comprise, e.g., one of the following, without being limited thereto: columns, capillaries, particles, membranes, filters, monoliths, fibers, pads, gels, slides, plates, cassettes, or any other format commonly used in chromatography and known to someone skilled in the art.

In one embodiment, the matrix is comprised of substantially spherical beads, for example Sepharose or Agarose beads. Matrices in particle form can be used as a packed bed or in a suspended form including expanded beds. In other embodiments of the invention, the solid support matrix is a membrane, for example a hydrogel membrane. In some embodiments, the affinity purification may involve a membrane as a matrix to which the affinity ligand for a dual GIP/GLP-1 receptor agonist as described herein is covalently bound. The solid support can also be in the form of a membrane in a cartridge.

**Use in affinity purification.** Some embodiments relate to the use of the novel affinity ligand for GIP/GLP-1 receptor agonist (Tirzepatide) of the present invention as disclosed herein, or a fusion protein of the present invention as described herein, or a solid phase support or affinity separation matrix of the present invention as described herein, for the affinity purification of a dual GIP/GLP-1 receptor agonist such as Tirzepatide, sample analysis for the detection of a dual GIP/GLP-1 receptor agonist such as Tirzepatide, or detection methods for the detection of a dual GIP/GLP-1 receptor agonist such as Tirzepatide.

Some embodiments relate to the use of the affinity separation matrix of the present invention comprising the novel affinity ligand for GIP/GLP-1 receptor agonist such as Tirzepatide of the present invention as disclosed herein, for the affinity purification of GIP/GLP-1 receptor agonists. Accordingly, the affinity separation matrix of the present invention comprising an affinity ligand of the present invention as disclosed herein is encompassed for use in the affinity purification of Tirzepatide.

As described herein, in particular embodiments, a solid phase support can be, or is preferably, an affinity separation matrix as described herein.

**Method for affinity purification of GIP/GLP-1 receptor agonist.** As described herein, affinity chromatography (also called affinity purification) makes use of specific binding interactions between molecules. Methods for immobilization of protein and methods for affinity chromatography are well-known in the field of protein purification and can be easily performed by a skilled person in this field using standard techniques and equipment.

The present invention provides a method for purification of a dual GIP/GLP-1 receptor agonist (Tirzepatide), the method comprising: (i) contacting a liquid containing a dual GIP/GLP-1 receptor agonist (Tirzepatide) with the affinity ligand of the present invention as described herein, or an affinity separation matrix of the present invention as described herein; (ii) purifying the dual GIP/GLP-1 receptor agonist (Tirzepatide); and (iii) obtaining the dual GIP/GLP-1 receptor agonist (Tirzepatide).

The present invention provides a method for purification of a dual GIP/GLP-1 receptor agonist (Tirzepatide), the method comprising: (i) providing a liquid that contains a dual GIP/GLP-1 receptor agonist (Tirzepatide); (ii) providing an affinity separation matrix of the present invention comprising at least one affinity ligand of the present invention as described herein coupled to said affinity separation matrix; (iii) contacting said affinity separation matrix of (ii) with the liquid of (i); (iv) purifying said dual GIP/GLP-1 receptor agonist (Tirzepatide) from said affinity separation matrix, and (v) obtaining the dual GIP/GLP-1 receptor agonist (Tirzepatide). In various embodiments, the method of affinity purification of the present invention may further comprise one or more washing steps after elution of a dual GIP/GLP-1 receptor agonist from the affinity separation matrix, by using a suitable solution such as sodium hydroxide.

In various embodiments, the method of affinity purification of the present invention may further comprise one or more steps for isolating the purified dual GIP/GLP-1 receptor agonist (Tirzepatide) via methods that are known to a person skilled in the art.

In some embodiments, the method of purification of the present invention involves a chromatography column containing a solid support (i.e. affinity separation matrix) to which the affinity ligand of the present invention as described herein is covalently bound. The affinity ligand of the present invention as described herein may be attached to a suitable solid support via conventional coupling techniques. Methods for immobilization of protein ligands to solid supports are well-known in the field of protein engineering and purification and can easily be performed by a skilled person in this field using standard techniques and equipment.

In various embodiments, the affinity ligands of the present invention as disclosed herein generated or obtained by any of the methods as described below are conjugated to a solid support. In some embodiments, the affinity ligand comprises an attachment site for site-specific covalent coupling of an affinity ligand of the present invention to a solid support. Specific attachment sites comprise without being limited thereto, natural amino acids, such as cysteine or lysine, which enable specific chemical reactions with a reactive group of the solid phase, or a linker between the solid phase and the protein, i.e., a affinity ligand of the present invention.

**Polynucleotides, vectors, host cells.** Some embodiments refer to a polynucleotide encoding the dual GIP/GLP-1 receptor agonist (Tirzepatide) affinity ligand of the present invention as described herein, or the fusion protein of the present invention as described herein.

The present invention encompasses an isolated polynucleotide or nucleic acid molecule encoding an affinity ligand for a dual GIP/GLP-1 receptor agonist (Tirzepatide) of the present invention as described herein. A further embodiment also encompasses proteins encoded by the polynucleotides.

Further provided is a vector, in particular an expression vector, comprising the isolated polynucleotide or nucleic acid molecule of the present invention encoding the affinity ligand of the present invention as described herein, as well as a host cell comprising the isolated polynucleotide or the expression vector of the present invention. For example, one or more polynucleotides, which encode the affinity ligand of the present invention as disclosed herein, may be expressed in a suitable host (cell) and the produced protein can be isolated. A vector means any molecule or entity (*e.g*., nucleic acid, plasmid, bacteriophage, or virus) that can be used for transfer of protein-encoding information into a host cell. Suitable vectors that may be applied are known in the art.

Suitable host cells include prokaryotes or eukaryotes, for example a bacterial host cell, a yeast host cell or a non-human host cell carrying a vector. A suitable host may be a microbial expression host that is capable to express the protein of the invention. Suitable bacterial expression host cells or systems are known in the art. Various mammalian or insect cell culture systems as known in the art can also be employed to express proteins.

Furthermore, an isolated cell comprising a polynucleotide or nucleic acid of the present invention, or a vector of the present invention, as disclosed herein, is provided.

**Method for producing dual GIP/GLP-1 receptor agonist (Tirzepatide).** The present invention provides a method for producing a dual GIP/GLP-1 receptor agonist (Tirzepatide), the method comprising: (i) providing a liquid containing a dual GIP/GLP-1 receptor agonist (Tirzepatide); (ii) contacting the liquid of (i) with an affinity ligand of the present invention as described herein, or contacting the liquid of (i) with the affinity separation matrix of the present invention as described herein; and (iii) purifying the dual GIP/GLP-1 receptor agonist (Tirzepatide).

In various embodiments, the method of producing a dual GIP/GLP-1 receptor agonist (Tirzepatide) may further comprise one or more washing steps.

**Isolating or purifying dual GIP/GLP-1 receptor agonist (Tirzepatide).** The present invention provides a method for isolating or purifying a dual GIP/GLP-1 receptor agonist (Tirzepatide) from a liquid or liquid sample, the method comprising:
(a) contacting a dual GIP/GLP-1 receptor agonist (Tirzepatide) affinity ligand of the present invention as described herein, or a multimeric dual GIP/GLP-1 receptor agonist (Tirzepatide) affinity ligand of the present invention as described herein, or a multimer of the present invention as described herein, or a fusion protein of the present invention as described herein, with a liquid or a liquid sample containing a dual GIP/GLP-1 receptor agonist (Tirzepatide);
(b) separating the dual GIP/GLP-1 receptor agonist (Tirzepatide) bound in step (a) to the dual GIP/GLP-1 receptor agonist affinity ligand, or to the fusion protein, respectively, from the liquid or the liquid sample;
(c) detaching or eluting said dual GIP/GLP-1 receptor agonist (Tirzepatide) from the affinity ligand, or the fusion protein, respectively.

The present invention further provides a method for isolating or purifying a dual GIP/GLP-1 receptor agonist (Tirzepatide) from a liquid or a liquid sample, the method comprising:
(a) contacting a solid phase support or affinity separation matrix of the present invention as described herein with a liquid or a liquid sample containing a dual GIP/GLP-1 receptor agonist (Tirzepatide), thereby binding of the dual GIP/GLP-1 receptor agonist (Tirzepatide) in the liquid or the liquid sample to the solid phase support or affinity separation matrix;
(b) detaching or eluting said dual GIP/GLP-1 receptor agonist (Tirzepatide) from the solid phase support or affinity separation matrix, respectively.

**Methods to analyze the presence of dual GIP/GLP-1 receptor agonist.** The present invention further provides the use of an affinity ligand for a dual GIP/GLP-1 receptor agonist (Tirzepatide) of the present invention as described herein, or an affinity matrix of the present invention as described herein in methods to determine or analyze the presence of a dual GIP/GLP-1 receptor agonist (Tirzepatide).

One embodiment refers to a method for analyzing the presence of a dual GIP/GLP-1 receptor agonist in a liquid or a liquid sample, the method comprising:
(a) contacting a dual GIP/GLP-1 receptor agonist (Tirzepatide) affinity ligand of the present invention as described herein, or a fusion protein of the present invention as described herein, or a solid phase support or affinity separation matrix of the present invention as described herein, with a liquid or a liquid sample containing a dual GIP/GLP-1 receptor agonist (Tirzepatide);
(b) determining the dual GIP/GLP-1 receptor agonist bound in step (a) to the dual GIP/GLP-1 receptor agonist (Tirzepatide) affinity ligand, or the fusion protein, or the solid phase support or affinity separation matrix, respectively;
(c) optionally detaching or eluting said dual GIP/GLP-1 receptor agonist (Tirzepatide) from the GIP/GLP-1 receptor (Tirzepatide) agonist affinity ligand, or the fusion protein, or the solid phase support or affinity separation matrix, respectively, prior to determining the dual GIP/GLP-1 receptor agonist (Tirzepatide).

**Method of quantification of dual GIP/GLP-1 receptor agonist (Tirzepatide).** The present invention further provides a method of quantification of a dual GIP/GLP-1 receptor agonist (Tirzepatide), the method comprising: (i) providing a liquid or a liquid sample that contains a dual GIP/GLP-1 receptor agonist (Tirzepatide); (ii) providing a matrix to which the affinity ligand of the present invention as described herein is covalently coupled; (iii) contacting said affinity separation matrix with the liquid under conditions that permit binding; (iv) eluting said dual GIP/GLP-1 receptor agonist (Tirzepatide); and (v) quantitating the amount of eluted dual GIP/GLP-1 receptor agonist (Tirzepatide). Methods to determine the presence of a dual GIP/GLP-1 receptor agonist (Tirzepatide) in liquids or in liquid samples might be quantitative or qualitative. Such methods are well known to the skilled person and can be selected, for instance but limited to, from the following methods that are well established in the art: enzyme-linked immunosorbent assay (ELISA), enzymatic reactions, surface plasmon resonance (SPR), or chromatography.

**Method of producing the affinity ligand of the invention.** The present invention further provides a method for the production of the affinity ligand of the present invention as described herein, the method comprising the step(s): (i) culturing a (suitable) host cell under conditions suitable for the expression of the affinity ligand so as to obtain said affinity ligand; and (ii) optionally isolating said affinity ligand. Suitable conditions for culturing a prokaryotic or eukaryotic host are well known to a person skilled in the art.

The affinity ligand of the present invention as described herein may be prepared by any conventional and well-known techniques such as plain organic synthetic strategies, solid phase-assisted synthesis techniques, or by commercially available automated synthesizers. They may also be prepared by conventional techniques, alone or in combination with conventional synthetic techniques.

The present invention provides a method for the preparation of the affinity ligand for a dual GIP/GLP-1 receptor agonist (Tirzepatide), as detailed above, said method comprising the steps: (i) providing a nucleic acid molecule encoding the affinity ligand of the present invention as described herein; (ii) introducing said nucleic acid molecule into an expression vector; (iii) introducing said expression vector into a host cell; (iv) culturing the host cell in a culture medium; (v) subjecting the host cell to culturing conditions suitable for expression thereby producing the affinity ligand; optionally (vi) isolating the polypeptide produced in step (v); and (vii) optionally conjugating the affinity ligand to a solid matrix as described above. In various embodiments of the present invention the production of the affinity ligand for a dual GIP/GLP-1 receptor agonist (Tirzepatide) of the present invention is performed by cell-free *in vitro* transcription and translation.

### EXAMPLES

The following Examples are provided for further illustration of the invention. The invention, however, is not limited thereto, and the following Examples merely show the practicability of the invention on the basis of the above description. For a complete disclosure of the invention reference is made also to the literature cited in the application which is incorporated completely into the application by reference.

### EXAMPLE 1. Purification of fusion protein 232902

Cells were solubilized in 50 mM BisTris, 1 mM EDTA, 100 mM NaCl, pH 6.5. After cell disruption an acidic acid concentration of 100 mM was adjusted in the lysate. Afterwards pH was adjusted to 2.0 followed by a centrifugation step (20 min, 30000xg). pH of supernatant was adjusted to 4.0 using 1 M Tris, pH 10. Supernatant was loaded on SP65 (Purolite) using an ÄKTAvant system (Cytiva) according to the manufacturer's instructions using acetic acid buffer at pH 4.0 (binding buffer: 50 mM acetic acid, 1 mM EDTA, pH 4.0 elution buffer: 50 mM acetic acid, 1 mM EDTA, 1M NaCl, pH 4.0). Fusion proteins were eluted in linear gradient from binding to elution buffer. Pooled protein was polished by size exclusion chromatography (Cytiva Superdex200 26/600) in 1xPBS. Protein concentrations were determined by absorbance measurement at 280 nm using the molar absorbent coefficient. Further analysis included RP-HPLC and SE-HPLC. Reversed phase chromatography (RP-HPLC) has been performed using an Agilent 1260 Infinity II System and a PLRP-S (5 µm, 300 Å) column (Agilent). The resulting purity of the fusion protein was >95 %. Analytic size exclusion chromatography (SE-HPLC) has been performed using an Agilent 1260 Infinity II System and a Superdex75 increase 5/150 GL or Superdex200 increase 5/150 GL (both Cytiva). The resulting purity was >95 %.

### EXAMPLE 2. Binding analysis of fusion proteins by SPR

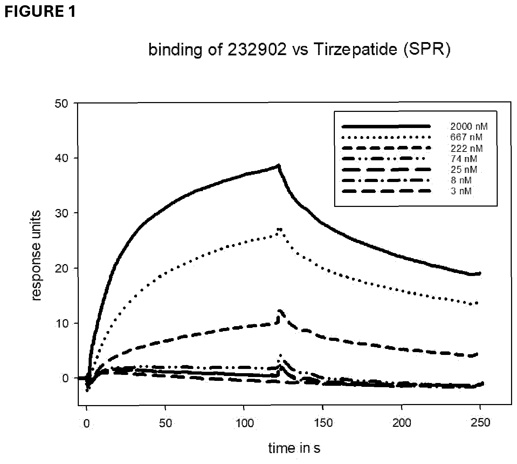

The purified fusion proteins were immobilized on a High Capacity Amine sensor chip (Bruker) using NHS/EDC activation with Sierra SPR-32 system (Bruker). The chip was equilibrated with SPR running buffer (PBS 0.05 % Tween, pH 7.3). Upon binding, target analyte was accumulated on the surface increasing the refractive index. This change in the refractive index was measured in real time and plotted as response or resonance units versus time. Tirzepatide and Semaglutide were applied to the chip in serial dilutions with a flow rate of 30 µl/min. The association was performed for 120 seconds and the dissociation for 120 seconds. After each run, the chip surface was regenerated with 30 µl regeneration buffer (10 mM glycine pH 2.0) and equilibrated with running buffer. Binding studies were carried out using Sierra SPR-32 system (Bruker); data evaluation was operated via Sierra Analyser software, provided by the manufacturer. The final analysis was done using the Langmuir 1:1 model (R_{Min}=0). Evaluated dissociation constants (K_{D}) were standardized against the immobilized protein and indicated. Shown is the change in refractive index measured in real time and plotted as response or resonance unit [RU] versus time [sec]. Fusion protein 232902 (SEQ ID NO: 3) binds specifically to the dual GIP/GLP-1 receptor agonist Tirzepatide with a K_{D} of 319 nM (see FIGURE 1). No binding vs. the GLP-1 receptor agoinst Semaglutide was observed. No binding vs. native GLP-1 was observed.

### EXAMPLE 3. Purification of Tirzepatide

**High coupling density to the affinity matrix.** The fusion protein was purified to homogeneity and immobilized at 20 mg per mL on activated Praesto Epoxy Jetted 50 (Purolite) according to the manufacturer's instructions, coupling conditions: 35 °C for 3 h, pH 9.5 per mL resin. Fusion protein 232902 (SEQ ID NO: 3) was successfully coupled to the epoxy activated resins, with achieved coupling density of about 15 mg/ml.

DBC10%. Coupled resin was packed into Omnifit 6.6/100 column (Diba). Tirzepatide was used as load (conc. 0.05 mg/ml in PBS pH 7.3). The sample was applied to the matrix comprising immobilized affinity ligand 232902 until 10 % target breakthrough at 6 min residence time. Unbound sample was washed off with 1xPBS, pH 7.3. The loaded sample was quantified and calculated as dynamic binding capacity DBC10 %. The binding capacity (DBC10) for affinity ligand 232902 was 0.7±0.4 mg/ml.

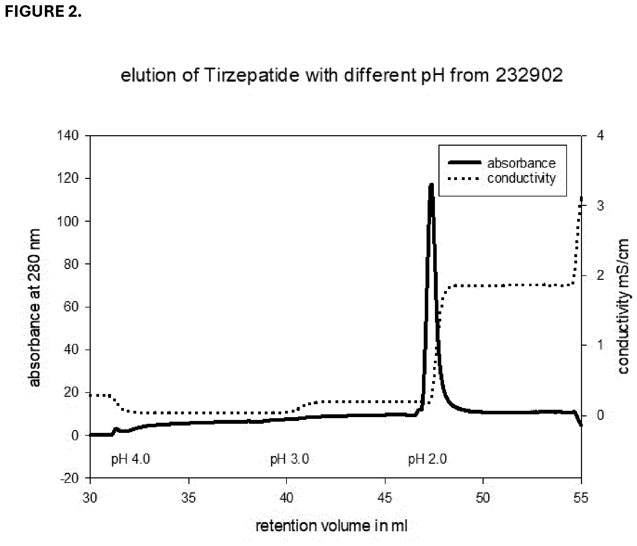

**Elution of Tirzepatide at pH 2.0.** Resin with coupled 232902 (SEQ ID NO: 3) was loaded with Tirzepatide (conc. 0.05 mg/ml; in 1x PBS, pH 7.3) with a contact time of 6 minutes. A wash step with 1xPBS pH 7.3 was included to remove unbound sample followed by a second wash step with 5 mM acetic acid pH 5.0. For determination of elution pH step gradients with 5 mM acetic acid and 20 % Hexylene glycol at pH 4.0, 5 mM acetic acid and 20 % Hexylene glycol at pH 3.0, 5 mM acetic acid and 20 % Hexylene glycol at pH 2.0 were introduced. Elution of Tirzepatide was possible with 5 mM acetic acid 20 % Hexylene glycol at pH 2.0 **(****Figure 2****).**

### Purification of Tirzepatide from E.coli supernatant:

*E.coli* BL21 (DE3) cells were grown over night in H15 Medium at 37°C and harvested via centrifugation. Cells were resuspended in 1xPBS and disrupted via high pressure homogenisator. The solution was centrifuged at 30000xg. Supernatant was spiked with 0.05 mg/ml Tirzepatide and loaded on coupled resin. Fractions were analyzed by SDS-PAGE. Tirzepatide was captured from *E.coli* supernatant and was successfully eluted with 5 mM acetic acid and 20% Hexylene glycol at pH 2.0. Elution fractions contained Tirzepatide in high purity whereas majority of impurities did not bound to the resin and were located in flowthrough.

### EXAMPLE 4. Validation of purified Tirzepatide

To confirm identity of eluted Tirzepatide binding vs GLP-1 receptor SPR measurements were used. Therefore GLP-1 receptor was immobilized on High Capacity Amine sensor chip (Bruker) using NHS/EDC activation with Sierra SPR-32 system (Bruker). The chip was equilibrated with SPR running buffer (PBS 0.05 % Tween, pH 7.3) and eluted Tirzepatide was injected with 250 nM. Eluted Tirzepatide showed binding vs. GLP-1 receptor.

### SEQUENCES:

SEQ ID NO: 1: GIP/GLP-1 receptor agonist binding moiety (comprised in fusion protein 232902)

AKFDEAQQAAFYEILHLPNLTEQQRNYFIQELRDDPSVSLEVLGKAQYLNDSQAIGYAMILSK
FDEAQQYAFYTILHLQNLTEEQRNAFIQSLRDDPSVSKEVLGEAWKLNLSQAPK

SEQ ID NO: 2: GIP/GLP-1 receptor agonist binding fusion protein (SEQ ID NO: 1 and SEQ ID NO: 5)

AKFDEAQQAAFYEILHLPNLTEQQRNYFIQELRDDPSVSLEVLGKAQYLNDSQAIGYAMILSK
FDEAQQYAFYTILHLQNLTEEQRNAFIQSLRDDPSVSKEVLGEAWKLNLSQAPKIAAQHDKI
QQAADKEILHLPNLTEEQRNKFRQSLRDDPSVSAEILAEAKKLNDAQAPK

SEQ ID NO: 3: GIP/GLP-1 receptor agonist binding fusion protein 232902 (SEQ ID NO: 1 and a dimer of SEQ ID NO: 5)

AKFDEAQQAAFYEILHLPNLTEQQRNYFIQELRDDPSVSLEVLGKAQYLNDSQAIGYAMILSK
FDEAQQYAFYTILHLQNLTEEQRNAFIQSLRDDPSVSKEVLGEAWKLNLSQAPKIAAQHDKI
QQAADKEILHLPNLTEEQRNKFRQSLRDDPSVSAEILAEAKKLNDAQAPKIAAQHDKIQQAA
DKEILHLPNLTEEQRNKFRQSLRDDPSVSAEILAEAKKLNDAQAPK

SEQ ID NO: 4: Non GIP/GLP-1 receptor agonist binding protein AQHDKIQQAAOKEILHLPNLTEEQRNKFRQSLRDDPSVSAEILAEAKKLNDAQAPK

SEQ ID NO: 5: Non GIP/GLP-1 receptor agonist binding protein IAAQHOKIQQAADKEILHLPNLTEEQRNKFRQSLRDDPSVSAEILAEAKKLNDAQAPK

SEQ ID NO: 6 Tirzepatide amino acid sequence YXEGTFTSDYSIXLDKIAQKAFVQWLIAGGPSSGAPPPS X is each alpha-aminoisobutyric acid (Aib).

## Claims

1. An affinity ligand for a dual glucose-dependent insulinotropic polypeptide (GIP) and glucagon-like peptide 1 (GLP-1) receptor agonist, comprising the amino acid sequence of SEQ ID NO: 1, or a sequence with at least 90 % sequence identity thereto, wherein the ligand exhibits a specific binding affinity for a dual GIP/GLP-1 receptor agonist.

2. The affinity ligand of claim 1, wherein the dual GIP/GLP-1 receptor agonist comprises the amino acid sequence of SEQ ID NO: 6, or a sequence with at least 90 % amino sequence identity thereto.

3. A fusion protein comprising:
(a) an affinity ligand for a dual GIP/GLP-1 receptor agonist according to claim 1 or 2; and
(b) a protein having at least 90 % sequence identity to SEQ ID NO: 4 and having no detectable binding affinity for a dual GIP/GLP-1 receptor agonist.

4. The fusion protein according to claim 3, which has at least 90% sequence identity to any one of SEQ ID NO: 2 or SEQ ID NO: 3.

5. The affinity ligand according to claim 1 or 2, or the fusion protein according to claim 3 or 4, which has a binding affinity for a dual GIP/GLP-1 receptor agonist of less than 500 nM, preferably a binding affinity for a dual GIP/GLP-1 receptor agonist comprising the amino acid sequence of SEQ ID NO: 6 of less than 500 nM.

6. A solid phase support, or an affinity separation matrix, comprising or having immobilized thereon, the affinity ligand for a dual GIP/GLP-1 receptor agonist according to any one of claims 1, 2, and 5, or the fusion protein according to any one of claims 3 to 5.

7. Use of the affinity ligand for a dual GIP/GLP-1 receptor agonist according to any one of claims 1, 2, and 5, or the fusion protein according to any one of claims 3 to 5, or the solid phase support or affinity separation matrix according to claim 6, in
(a) affinity purification of a dual GIP/GLP-1 receptor agonist;
(b) sample analysis for the detection of a dual GIP/GLP-1 receptor agonist; or
(c) detection methods for the detection of a dual GIP/GLP-1 receptor agonist.

8. A polynucleotide encoding the affinity ligand for a dual GIP/GLP-1 receptor agonist according to any one of claims 1, 2, and 5, or the fusion protein according to any one of claims 3 to 5.

9. A method of isolating or purifying a dual glucose-dependent insulinotropic polypeptide (GIP) and glucagon-like peptide 1 (GLP-1) receptor agonist from a liquid, the method comprising:
(a) contacting the affinity ligand for a dual GIP/GLP-1 receptor agonist according to any one of claims 1, 2, and 5, or the fusion protein according to any one of claims 3 to 5, with a liquid containing a dual GIP/GLP-1 receptor agonist;
(b) separating the dual GIP/GLP-1 receptor agonist bound in step (a) to the affinity ligand for a dual GIP/GLP-1 receptor agonist, or to the fusion protein, respectively, from the liquid;
(c) detaching or eluting said dual GIP/GLP-1 receptor agonist from the affinity ligand for a dual GIP/GLP-1 receptor agonist, or the fusion protein, respectively.

10. A method of isolating or purifying a dual GIP/GLP-1 receptor agonist from a liquid, the method comprising:
(a) contacting the solid phase support or affinity separation matrix according to claim 6 with a liquid containing a dual GIP/GLP-1 receptor agonist, thereby binding of a dual GIP/GLP-1 receptor agonist in the liquid to the solid phase support or affinity separation matrix;
(b) detaching or eluting said dual GIP/GLP-1 receptor agonist from the solid phase support or affinity separation matrix, respectively.

11. A method of determining the presence of a dual GIP/GLP-1 receptor agonist in a liquid, the method comprising:
(a) contacting the affinity ligand for a dual GIP/GLP-1 receptor agonist according to any one of claims 1, 2, and 5, or the fusion protein according to any one of claims 3 to 5, or the solid phase support or affinity separation matrix according to claim 6, with a liquid containing a dual GIP/GLP-1 receptor agonist;
(b) determining the GIP/GLP-1 receptor agonist bound in step (a) to the affinity ligand for a dual GIP/GLP-1 receptor agonist, or the fusion protein, or the solid phase support or affinity separation matrix, respectively;
(c) optionally detaching or eluting said dual GIP/GLP-1 receptor agonist from the affinity ligand for a dual GIP/GLP-1 receptor agonist, or the fusion protein, or the solid phase support or affinity separation matrix, respectively, prior to determining the dual GIP/GLP-1 receptor agonist.
